# EUROPEAN PATENT SPECIFICATION

(11) **EP 4 180 792 B1**
(45) Date of publication and mention of the grant of the patent: **12.06.2024**
(21) Application number: 21207382.9
(22) Date of filing: 10.11.2021
(51) Int. Cl.: G01N 1/14, G01N 33/18, B01D 35/143, G01F 11/12

(54) **A METHOD FOR DETERMINING A SAMPLE FILTER CLOGGING CONDITION VALUE**
VERFAHREN ZUR BESTIMMUNG EINES VERSTOPFUNGSZUSTANDSWERTS EINES PROBENFILTERS
PROCÉDÉ PERMETTANT DE DÉTERMINER UNE VALEUR DE L'ÉTAT D'ENCRASSEMENT D'UN FILTRE D'ÉCHANTILLONS

(43) Date of publication of application: 17.05.2023
(73) Proprietor: Hach Lange GmbH, 14163 Berlin (DE)
(72) Inventor: GOLITZ, Andreas, 47475 Kamp-Lintfort (DE); BATTEFELD, Manfred, 40211 Düsseldorf (DE)
(74) Representative: terpatent Patentanwälte und European Patent Attorneys ter Smitten Eberlein -Van Hoof

(56) References cited:
- WO-A1-2021/018404
- US-A1- 2016 200 607

## Description

The invention refers to a method for determining a sample filter clogging condition value of an immersion filter probe of a process water analyzer arrangement.

A typical process water analyzer arrangement is disclosed in WO 2021 018 404 A1. The process water analyzer arrangement is provided with a stationary immersion filter probe being immersed into a water basin and is provided with a land-based analyzing unit comprising a sample pump and a physical analyzer, e.g. a photometric analyzer, for analyzing the filtered water sample. The sample pump is typically provided as a peristaltic hose pump because this pump type is principally robust against clogging, and the pump hose being the only pump part coming into direct contact with the pumped water can easily be substituted.

The pumping performance depends on the filter membrane clogging condition as well as on the pump hose condition. However, if no pressure sensor is provided, it is not possible to directly determine the transmembrane pressure which would be an indication for the filter clogging condition of the filter membrane.

Reference is also made to US 2016/200607 A1 which discloses a waste water treatment plant incorporating a membrane bioreactor (MBR) system comprising among others a reactor tank, and a membrane filter unit submerged in a side tank of the reactor tank and fluidly connected to a permeate storage tank via a motor-driven peristaltic permeate feed pump. Clogging of the membrane filter unit is detected by reduced flow rate measured by rotameters or by transmembrane pressure drop (TMP) measured by a pressure transducer.

It is an object of the invention to provide a simple method for determining a sample filter membrane clogging condition value of an immersion filter probe being fluidically in-line with a peristaltic hose sample pump of a process water analyzer arrangement.

This object is solved with a method for determining a sample filter membrane clogging condition value with the features of claim 1.

The method according to the invention refers to a process water analyzer arrangement comprising an immersion filter probe being arranged within a water basin and an analyzing unit comprising a water sample analyzer for analyzing a water sample filtered by the immersion filter probe. The preferably land-based analyzing unit comprises a bidirectional peristaltic hose sample pump for pumping the water sample from the immersion filter probe to the water sample analyzer. The peristaltic hose sample pump is fluidically arranged between the filter probe and the water sample analyzer, and can be driven in both fluidic directions, namely in forward and in backward direction.

The sample pump comprises a flexible elastic peristaltic pump hose, a pump motor and a peristaltic pump rotor for providing a peristaltic deformation of the deformable flexible pump hose. The pump motor is controlled by a motor speed control so that the motor speed is known to the motor speed control. After starting the pump, the total number of pump rotor rotations is always known to the motor speed control.

The arrangement comprises a bidirectional water flow sensor unit being arranged in-line with the filter probe, the sample pump and the water sample analyzer. Preferably, the flow sensor unit is arranged downstream of the sample pump and upstream of the sample analyzer. The flow sensor precisely detects and determines a total flow volume in the range of several milliliters and with an accuracy of preferably at least 0.1 ml. The flow sensor can be used in forward pumping direction and in backward pumping direction.

The immersion filter probe comprises a sample filter membrane being proximally mechanically supported by a support structure so that the sample filter membrane is perfectly mechanically supported and is not movable when the water is being pumped or sucked in forward direction through the sample filter membrane to the sample pump. The sample filter membrane is distally not perfectly mechanically supported and is bulged with a membrane distal bulging volume when water is pumped in backward direction from the sample pump to the filter probe. Since even a new sample filter membrane has a flow resistance of, for example, 100 mbar at a standard water flow rate in backward direction, the sample filter membrane is bulged at the beginning of a backward pumping action until the membrane distal bulging movement is finished because the filter membrane is completely tensed. As soon as the filter membrane is tensed, the water pumped in backward direction flows in backward direction through the filter membrane or the filter membrane breaks. In practice, the membrane distal bulging volume is only several milliliters and can be up to 20 ml. Preferably, the typical bulging volume of the filter membrane is less than 10 ml.

The process water analyzer arrangement comprises an electronic filter clogging condition determination module for determining a sample filter clogging condition value of the sample filter membrane. The clogging condition determination module is informationally connected to the sample pump and to the flow sensor unit by suitable signal connections. The clogging condition determination module can actively control the action of the sample pump.

The filter clogging condition determination module provides the following method steps:
The filter clogging condition determination module drives the sample pump with a known rotational speed for pumping a defined filtration volume flowing in the forward direction through the filter membrane, the pumping of the correct defined filtration volume being detected and controlled by the water flow sensor unit. After the defined filtration volume has been pumped through the filter membrane, the filtration motor rotations value for pumping the defined filtration volume is memorized in a suitable memory of the filter clogging condition determination module. The filtration motor rotations value is provided by the motor speed control.

Before or after the latter pump action, the sample pump is driven for pumping a defined non-filtration volume which volume is detected and controlled by the water flow sensor unit. The defined total non-filtration volume is at least minimally smaller than the membrane distal bulging volume so that there is no relevant transmembrane water flow during this method step. The motor rotations value for pumping the defined non-filtration volume is memorized by the filter clogging condition determination value module. Preferably, the non-filtration volume is less than 10 ml.

Generally, the pumping of the defined non-filtration volume can be provided in forward or in backward flow direction. If the filter membrane is completely bulged before the latter method step is started, the latter method step of pumping a defined non-filtration volume is provided in forward pumping direction. If the filter membrane is completely non-bulged and is completely in contact with the membrane support structure, the latter method step is provided in backward pumping direction. However, the sample pump must be of the bidirectional type.

Preferably, the sample pump is pumping in forward direction for pumping the defined filtration volume and is pumping in backward direction for pumping the defined non-filtration volume. The forward pumping direction generates a water flow from the filter probe to the sample pump, the backward pumping direction generates a water flow from the sample pump to the filter probe.

Preferably, no pressure sensor is provided fluidically between the filter probe and the sample pump.

Finally, the filter clogging condition determination module determines a sample filter clogging condition value based on the memorized filtration motor rotations value and the memorized non-filtration motor rotations value. The two said rotations values allow an indication of the sample filter clogging condition because the total number of motor rotations needed for pumping the non-filtration volume is substantially effected by the physical condition of the peristaltic hose of the sample pump, but is not substantially effected by the clogging condition of the filter membrane. The total number of motor rotations needed for pumping the defined filtration volume flowing through the filter membrane without bulging the filter membrane is effected by both, the physical condition of the peristaltic hose of the sample pump and the clogging condition of the filter membrane. These physical interrelations allow to determine a sample filter clogging value without using any pressure sensor for directly detecting a transmembrane pressure of the filter membrane.

Preferably, the determination of the sample filter clogging condition value is based on the basic principle that the filter clogging condition value is the better the smaller the difference is between the filtration motor rotations value and the non-filtration motor rotations value or the smaller the ratio is of the filtration motor rotations value and the non-filtration motor rotations value. A good filter clogging condition value means that the filter membrane has a relatively low fluidic resistance in forward flow direction.

Preferably, the defined filtration volume is substantially equal or absolutely equal to the defined non-filtration volume.

Preferably, the water flow sensor unit is provided downstream of the sample pump. The water flow sensor unit can be provided with two sensor elements and determines the sample water travel time between the two sensor elements. Even more preferably, the flow sensor unit comprises a vertical measuring container with two water level sensor elements being arranged in a vertical distance to each other. Since the analyzing unit of known process water analyzer arrangements generally are provided with a very precise vertical measuring container with two water level sensor elements, no additional elements are needed for providing a filter clogging condition determination module according to the invention.

Preferably, the pump motor driving the pump rotor is a step motor. A step motor allows a perfect control and recording of the pump rotor rotations, and allows to simply record and count the pump motor rotations and the pump rotor rotations without an additional motor sensor.

Preferably, a hydrostatic height memory is provided as part of the filter clogging condition determination module for memorizing the hydrostatic pump height value between the sample pump and the water surface in the water basin with the immersed immersion filter probe. The total height difference between the immersion filter probe and the sample pump effects the deformation of the pump hose during a pumping action so that the knowledge of the height difference is essential for a correct determination of the sample filter clogging condition value.

Preferably, the arrangement determines a hose condition value on the basis of the lately memorized non-filtration motor rotations value, for example, in relation to the same value of a new pump hose. If the determined non-filtration motor rotations value or the relation of the value with the corresponding value for a new hose exceeds a predefined difference or rate, a signal indicating a worn-out pump hose is generated and issued.

An embodiment of the invention is described with reference to the drawings, wherein
figure 1 shows schematically a process water analyzer arrangement with an immersion filter probe in a water basin and a land-based analyzing unit, and
figure 2 shows an evaluation diagram for determining a sample filter clogging condition value.

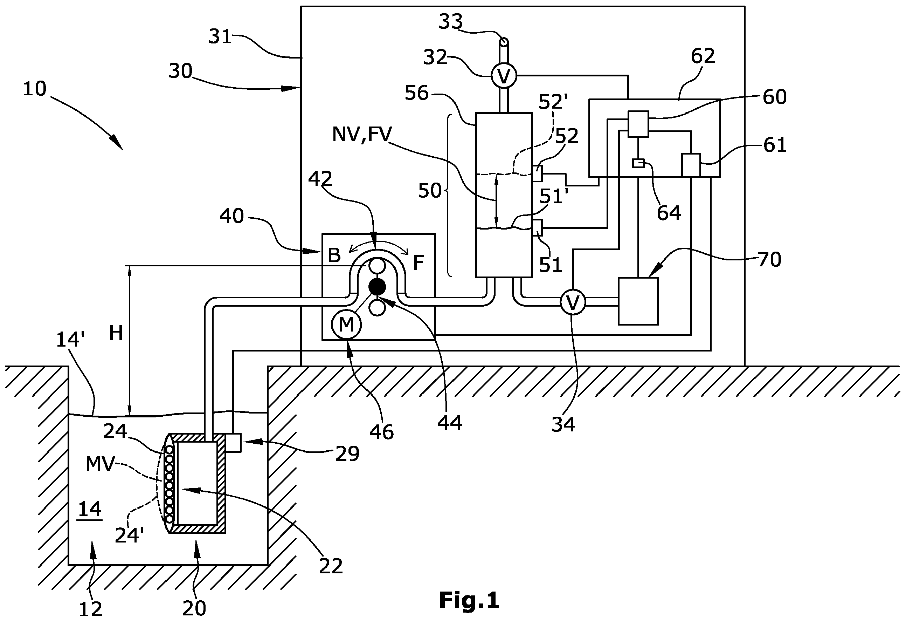

Figure 1 schematically shows a typical process water analyzer arrangement 10 being arranged at a water treatment plant comprising a water basin 12 filled with water 14. The process water analyzer arrangement 10 substantially comprises an immersion filter probe 20 arranged in the water basin 12 and a land-based analyzing unit 30. The analyzing unit 30 comprises within an analyzing unit housing 31, a bidirectional sample pump 40, a bidirectional water flow sensor unit 50, a filter clogging condition determination module 60 and a water sample analyzer 70.

The water sample analyzer 70 is a photometer determining the transmission or the absorption of a water sample being treated by a suitable color-changing reagent and being pumped by the sample pump 40 to the sample analyzer 70.

The water basin 12 is always filled with water 14 defining a water level surface 14' which is substantially at a constant level within the water basin 12. The immersion filter probe 20 is statically arranged in the water basin 12 at a constant vertical position and is provided with a sample filter membrane 24 being proximally supported by a membrane support structure 22 which can be, for example, a quadratic wire grid raster. The sample filter membrane 24 is completely immersed into the water 14. The distal side of the sample filter membrane 24 is not mechanically supported so that the membrane 24 can be bulged if the proximal membrane pressure is higher than the distal membrane pressure. The bulged sample filter membrane 24' defines a membrane distal bulging volume MV between the membrane support structure 22 and the bulged filter membrane 24'. The maximum membrane distal bulging volume MV is about 10 ml. The immersion filter probe 20 is provided with an external temperature sensor 29 for sensing the temperature of the basin water 14.

The bidirectional peristaltic hose sample pump 40 comprises a pump motor 46 being a speed-controlled step motor, a peristaltic pump rotor 44 and a flexible elastic peristaltic pump hose 42 being continuously deformed by the cooperating rotating pump rotor 44. The sample pump 40 is fluidically arranged between the filter probe 20 and the water flow sensor unit 50, and can be driven in forward direction F for pumping a water sample to the water flow sensor unit 50 and the water sample analyzer 70 and in backward direction B for pumping water from the water flow sensor unit 50 back to the immersion filter probe 20. One full rotation of the pump rotor 44 pumps, under perfect conditions, 200 µl water per rotation, and pumps under worse conditions, less than 100 µl per rotation. The total pumping rate depends, among other factors, on the clogging of the sample filter membrane 24 and on the physical condition of the pump hose 42.

The pump motor 46 is controlled by a motor speed control 61 being a part of a of an electronic analyzer control unit 62.

No pressure sensor is provided fluidically between the filter probe 20 and the sample pump 40. The sample pump 40 is arranged in a hydrostatic pump height with a constant height value H above the basin water level 14' which value is memorized in a hydrostatic height memory 64 being a part of the analyzer control unit 62. After the water analyzer arrangement 10 has been mounted to the corresponding plant site, the hydrostatic pump height value H is entered manually into the hydrostatic height memory 64.

The bidirectional water flow sensor unit 50 comprises a vertical cylindrical measuring container 56 with two separate water level sensor elements 51, 52 allowing to precisely detect a defined low water level 51' and a defined high water level 52' of the water within the measuring container 56. The container interior is fluidically connectable via a venting valve 32 to an atmospheric venting opening 33 for allowing the water level within the sensor unit container 56 to rise or to fall. The volume difference between the two water levels 51', 52' is, for example, exactly 2.00 ml which is exactly the value for a defined filtration volume FV and a defined non-filtration volume NV.

A sample valve 34 is provided in the fluid line between the flow sensor unit 50 and the water sample analyzer 70, which sample valve 34 is closed during the filling or discharging the measuring container 56.

After a filter condition determination has been triggered automatically or manually, for example once a day, the filter clogging condition determination module 60 provides the following method steps:
For preparing the determination process, the filter clogging condition determination module 60 causes the sample pump 42 to rotate in forward direction F to ensure that the sample filter membrane 24 is completely in direct mechanical contact with the membrane support structure 22 and is not bulged. The container 56 of the water flow sensor unit 50 is filled precisely up to the upper water level 52' detected by the upper water level sensor element 52. The forward pumping action is completely stopped as soon as the upper water level 52' has been arrived detected by the upper water level sensor element 52.

In the first determination method step, the sample pump 40 is driven by the motor control 61 speed-controlled in backward direction B, and the total number of rotations of the pump rotor 44 is counted and accumulated with a resolution of 72°/360° until the lower water level 51' is reached which is detected by the lower water level sensor element 51. This means that a defined non-filtration volume NV of precisely 2.00 ml is pumped backwards to the filter probe 20. Since the filter membrane 24 has even in an unused new condition a relevant flow resistance for water, the water pumped backwards into the filter probe causes the filter membrane 24' to bulge but not to let the water pass the filter membrane 24'. The pumping performance of the sample pump 40 is not effected by the clogging condition of the filter membrane 24 but is effected by the known hydrostatic height H and the physical condition of the pump hose 42.

The total number of motor rotor rotations needed for pumping the defined non-filtration volume NV is memorized in the filter clogging condition determination module 60 as a defined non-filtration motor rotations value NR.

Before the following measurement step is provided, the sample pump 40 is caused to pump in forward direction F and the valves 32, 34 are switched to pump a volume of, for example 10 ml in forward direction F to the analyzer to ensure that the filter membrane 24, again, is in direct contact with the filter membrane support structure 22 and is not bulged anymore. The water in the water flow sensor unit 50 remains precisely at the lower water level 51' detected by the lower sensor element 51.

The venting valve 32 is opened and the sample valve 34 is closed. The filter clogging condition determination module 60 now causes the sample pump 40 to pump in forward direction F the defined filtration volume FV of 2.00 ml which is detected by the upper sensor element 52, and to accumulate the filtration motor rotations value FR which is the total number of motor rotor rotations which is necessary to pump the defined filtration volume FV through the filter membrane 24. The pumping performance and the filtration motor rotations value FR depend on the filter clogging condition of the filter membrane 24, the known water temperature T detected by the temperature sensor 29, the known hydrostatic height H and the physical condition of the pump hose 42.

As a result, the filter clogging condition determination module 60 can now determine the sample filter clogging condition value C based on the known filtration motor rotations value FR, the known water Temperature T and the known non-filtration motor rotations value NR.

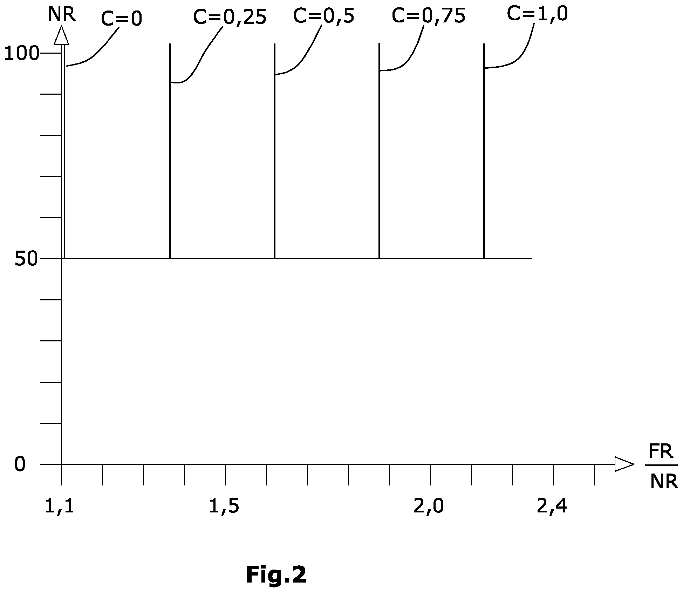

Figure 2 shows a very schematic diagram which is memorized in the filter clogging condition determination module 60. As can be seen in the diagram of figure 2, the sample filter clogging condition value C is the better, the smaller the quotient FR/NR is. A condition value of C=0 is perfect, a condition value of C=1.0 indicates a too high clogging of the filter membrane 24 so that the filter membrane 24 should be cleaned or substituted.

A motor rotations value of 5 corresponds with one full rotor rotation of 360°. If the non-filtration motor rotations value NR is above 100, which is 200% of the initial non-filtration motor rotations value 50 of a new pump hose, the peristaltic pump hose 42 is physically not in a good condition anymore and should be substituted. The relation of the latter two values is signalized as a pump hose condition value CH.

## Claims

1. A method for determining a sample filter clogging condition value (C) of an immersion filter probe (20) of a process water analyzer arrangement (10),
the arrangement (10) comprising the immersion filter probe (20) arranged in a water basin (12) and an analyzing unit (30) comprising a water sample analyzer (70) for analyzing a water sample filtered by the immersion filter probe (20),
the arrangement (10) comprising a bidirectional peristaltic hose sample pump (40) for pumping a water sample from the immersion filter probe (20) to the water sample analyzer (70), the sample pump (40) comprising a peristaltic pump hose (42) and a pump motor (46) driving a peristaltic pump rotor (44), the pump motor (46) being speed-controlled by a motor speed control (61),
the immersion filter probe (20) comprising a sample filter membrane (24) being proximally mechanically supported by a support structure (22) and having a membrane distal bulging volume (MV),
the arrangement (10) comprising a bidirectional water flow sensor unit (50) being arranged in-line with the sample pump (40) and the water sample analyzer (70), and
the arrangement (10) comprising a filter clogging condition determination module (60) for determining the sample filter clogging condition value (C) of the sample filter membrane (24),
with the method steps provided by the filter clogging condition determination module (60):
driving the sample pump (40) with a known rotational speed for pumping a defined filtration volume (FV) flowing in forward direction through the filter membrane (24) and being detected by the water flow sensor unit (50),
memorizing the filtration motor rotations value (FR) generated by the motor speed control (61) for pumping the defined filtration volume (FV),
driving the sample pump (40) for pumping a defined non-filtration volume (NV) detected by the water flow sensor unit (50), wherein the non-filtration volume (NV) is smaller than the membrane distal bulging volume (MV),
memorizing the motor rotations value (NR) generated by the motor speed control (61) for pumping the defined non-filtration volume (NV), and
determining the sample filter clogging condition value (C) based on the filtration motor rotations value (FR) and the non-filtration motor rotations value (NR).

2. The method of claim 1, wherein the sample pump (40) is pumping in forward direction (F) for pumping the defined filtration volume (FV) and is pumping in backward direction (B) for pumping the defined non-filtration volume (NV).

3. The method of any one of the preceding claims, wherein the defined filtration volume (FV) is substantially equal to the defined non-filtration volume (NV).

4. The method of any one of the preceding claims, wherein the water flow sensor unit (50) is provided downstream of the sample pump (40).

5. The method of any one of the preceding claims, wherein the water flow sensor unit (50) is provided with two sensor elements (51, 52) and determines a travel time (T) between the two sensor elements (51, 52).

6. The method of any one of the preceding claims, wherein the flow sensor unit (50) comprises a vertical measuring container (56) with two water level sensor elements (51, 52).

7. The method of any one of the preceding claims, wherein the defined non-filtration volume (NV) is less than 10 ml.

8. The method of any one of the preceding claims, wherein the filter membrane (24) is distally not mechanically supported.

9. The method of any one of the preceding claims, wherein no pressure sensor is provided fluidically between the filter probe (20) and the sample pump (40).

10. The method of any one of the preceding claims, wherein the pump motor (46) is a step motor.

11. The method of any one of the preceding claims, wherein a hydrostatic height memory (64) for memorizing the hydrostatic pump height value (H) between the sample pump (40) and the water surface (40') in the water basin (12), wherein the determining of the sample filter clogging condition value (C) includes the hydrostatic pump height (H).

12. The method of any one of the preceding claims, wherein the sample filter clogging condition value (C) is the better the smaller the ratio is of the filtration motor rotations value (FR) and the non-filtration motor rotations value (NR).

13. The method of any one of the proceeding claims, wherein the arrangement (10) determines a pump hose condition value (CH) on the basis of the non-filtration motor rotations value (NR).

## Patentansprüche

1. Ein Verfahren zur Bestimmung eines Probenfilter-Verstopfungszustandswertes (C) einer Tauchfiltersonde (20) einer Prozess-Wasseranalysator-Anordnung (10),
wobei die Anordnung (10) die in einem Wasserbecken (12) angeordnete Tauchfiltersonde (20) und eine Analyseeinheit (30) aufweist, die einen Wasserprobenanalysator (70) zum Analysieren einer durch die Tauchfiltersonde (20) gefilterten Wasserprobe aufweist,
die Anordnung (10) eine bidirektionale Probenahmepumpe (40) mit peristaltischem Schlauch zum Pumpen einer Wasserprobe von der Tauchfiltersonde (20) zu dem Wasserprobenanalysator (70) aufweist, wobei die Probenahmepumpe (40) einen peristaltischen Pumpenschlauch (42) und einen Pumpenmotor (46) aufweist, der einen peristaltischen Pumpenrotor (44) antreibt, wobei der Pumpenmotor (46) durch eine Motordrehzahlregelung (61) drehzahlgeregelt ist,
die Tauchfiltersonde (20) eine Probenfiltermembran (24) aufweist, die proximal mechanisch von einem Haltemittel (22) gehalten wird und ein distales Membran-Auswölbungsvolumen (MV) aufweist,
die Anordnung (10) eine bidirektionale Wasserdurchfluss-Sensoreinheit (50) aufweist, die in Reihe mit der Probenahmepumpe (40) und dem Wasserprobenanalysator (70) angeordnet ist, und
die Anordnung (10) ein Filterverstopfungs-Bestimmungsmodul (60) zur Bestimmung des Probenfilter-Verstopfungszustandswertes (C) der Probenfiltermembran (24) aufweisend ist,
mit den Verfahrensschritten, die das Filterverstopfungs-Bestimmungsmodul (60) ausführt:
Steuern der Probenahmepumpe (40) mit einer bekannten Drehzahl zum Pumpen eines definierten Filtrationsvolumens (FV), das in Vorwärtsrichtung durch die Filtermembran (24) fließt und von der Wasserdurchfluss-Sensoreinheit (50) erfasst wird,
Speichern des von der Motordrehzahlregelung (61) generierten Filtrationsmotor-Drehzahlwertes (FR) zum Pumpen des definierten Filtrationsvolumens (FV),
Ansteuern der Probenahmepumpe (40) zum Pumpen eines definierten Nicht-Filtrationsvolumens (NV), das von der Wasserdurchfluss-Sensoreinheit (50) erfasst wird, wobei das Nicht-Filtrationsvolumen (NV) kleiner ist als das distale Membran-Auswölbungsvolumen (MV),
Speichern des Motorumdrehungswertes (NR), der von der Motordrehzahlregelung (61) zum Pumpen des definierten Nicht-Filtrationsvolumens (NV) erzeugt wird, und
Bestimmen des Probenfilter-Verstopfungszustandswertes (C) auf der Grundlage des Filtrations-Motorumdrehungswertes (FR) und des NichtFiltrations-Motorumdrehungswertes (NR).

2. Das Verfahren nach Anspruch 1, wobei die Probenahmepumpe (40) in Vorwärtsrichtung (F) pumpt, um das definierte Filtrationsvolumen (FV) zu pumpen, und in Rückwärtsrichtung (B) pumpt, um das definierte Nicht-Filtrationsvolumen (NV) zu pumpen.

3. Das Verfahren nach einem der vorangehenden Ansprüche, wobei das definierte Filtrationsvolumen (FV) im Wesentlichen gleich dem definierten Nicht-Filtrationsvolumen (NV) ist.

4. Das Verfahren nach einem der vorangehenden Ansprüche, wobei die Wasserdurchfluss-Sensoreinheit (50) stromabwärts der Probenahmepumpe (40) vorhanden ist.

5. Das Verfahren nach einem der vorangehenden Ansprüche, wobei die Wasserdurchfluss-Sensoreinheit (50) zwei Sensorelemente (51, 52) aufweist und eine Laufzeit (T) zwischen den beiden Sensorelementen (51, 52) bestimmt.

6. Das Verfahren nach einem der vorangehenden Ansprüche, wobei die Wasserdurchfluss-Sensoreinheit (50) einen vertikalen Messbehälter (56) mit zwei Wasserstands-Sensorelementen (51, 52) aufweist.

7. Das Verfahren nach einem der vorangehenden Ansprüche, wobei das definierte Nicht-Filtrationsvolumen (NV) weniger als 10 ml beträgt.

8. Das Verfahren nach einem der vorangehenden Ansprüche, wobei die Filtermembran (24) distal nicht mechanisch gestützt ist.

9. Das Verfahren nach einem der vorangehenden Ansprüche, wobei fluidisch zwischen der Filtersonde (20) und der Probenahmepumpe (40) kein Drucksensor vorhanden ist.

10. Das Verfahren nach einem der vorangehenden Ansprüche, wobei der Pumpenmotor (46) ein Schrittmotor ist.

11. Das Verfahren nach einem der vorangehenden Ansprüche, wobei ein hydrostatischer Höhenspeicher (64) zum Speichern des hydrostatischen Pumpenhöhenwertes (H) zwischen der Probenahmepumpe (40) und der Wasseroberfläche (40') im Wasserbecken (12) vorgesehen ist, wobei die Bestimmung des Probenfilter-Verstopfungszustandswertes (C) die hydrostatische Pumpenhöhe (H) berücksichtigt.

12. Das Verfahren nach einem der vorangehenden Ansprüche, wobei der Probenfilter-Verstopfungszustandswert (C) um so günstiger ist, je kleiner das Verhältnis zwischen dem Filtrations-Motorumdrehungswert (FR) und dem Nichtfiltrations-Motorumdrehungswert (NR) ist.

13. Das Verfahren nach einem der vorhergehenden Ansprüche, wobei die Anordnung (10) einen Pumpenschlauch-Zustandswert (CH) auf der Basis des Nichtfiltrations-Motorumdrehungswertes (NR) bestimmt.

## Revendications

1. Une méthode pour déterminer une valeur de condition de colmatage du filtre à échantillon (C) d'une sonde à filtre immergé (20) d'un arrangement d'analyseur d'eau d'un processus d'analyse (10), l'arrangement (10) comprenant la sonde à filtre immergé (20) disposée dans un bassin d'eau (12) et une unité d'analyse (30) comprenant un analyseur d'échantillon d'eau (70) pour analyser un échantillon d'eau filtré par la sonde à filtre immergé (20),
l'arrangement (10) comprend une pompe à échantillon bidirectionnelle à tuyau péristaltique (40) pour pomper un échantillon d'eau de la sonde de filtre à immergé (20) vers l'analyseur d'échantillons d'eau (70), la pompe à échantillon (40) comprenant un tuyau de pompe péristaltique (42) et un moteur de pompe (46) entraînant un rotor de pompe péristaltique (44), le moteur de pompe (46) étant contrôlé en vitesse par un contrôle de vitesse de moteur (61),
la sonde filtre immergé (20) comprend une membrane de filtre à échantillon (24) étant proximalement supportée mécaniquement par une structure de support (22) et ayant un volume renflé distal de la membrane (MV),
l'arrangement (10) comprend une unité de senseur de flux d'eau bidirectionnel (50) étant disposée en ligne avec la pompe à échantillon (40) et l'analyseur d'échantillon d'eau (70), et.
l'arrangement (10) comprend un module de détermination de la condition de colmatage du filtre (60) permettant de déterminer la valeur de la condition de colmatage du filtre à échantillon (C) de la membrane de filtre à échantillon (24),
avec les marches de la méthode fournies par le module de détermination de la condition de colmatage du filtre (60) :
entraîner la pompe à échantillon (40) avec une vitesse de rotation connue pour pomper un volume de filtration tel que défini (FV) s'écoulant en direction avant à travers la membrane de filtre (24) et étant détecté par l'unité de senseur de flux d'eau (50),
mémoriser la valeur de rotations du moteur de filtration (FR) générée par le contrôle de vitesse moteur (61) pour pomper le volume de filtration tel que défini (FV),
entraînant la pompe à échantillon (40) pour le pompage d'un volume de non-filtration défini (NV) détecté par l'unité de senseur de flux d'eau (50), dans lequel le volume de non-filtration (NV) est plus petit que le volume de renflement distal de la membrane (MV),
mémoriser la valeur de rotations moteur (NR) générée par le contrôle de vitesse moteur (61) pour pomper le volume de non-filtration défini (NV), et
déterminer la valeur de condition de colmatage du filtre à échantillon (C) en fonction de la valeur de rotations du moteur de filtration (FR) et de la valeur de rotations du moteur de non-filtration (NR).

2. La méthode de la revendication 1, dans laquelle la pompe à échantillon (40) pompe dans le sens avant (F) pour pomper le volume de filtration défini (FV) et pompe dans le sens arrière (B) pour pomper le volume de non-filtration défini (NV).

3. La méthode de l'une quelconque des revendications précédentes, dans laquelle le volume de filtration défini (FV) est substantiellement égal au volume de non-filtration défini (NV).

4. La méthode de l'une quelconque des revendications précédentes, dans laquelle l'unité de senseur de flux d'eau (50) est fournie en aval de la pompe à échantillon (40).

5. La méthode de l'une quelconque des revendications précédentes, dans laquelle l'unité de senseur de flux d'eau (50) est fournie avec deux éléments de capteur (51, 52) et détermine un temps de parcours (T) entre les deux éléments de capteur (51, 52).

6. La méthode de l'une quelconque des revendications précédentes, dans laquelle l'unité de senseur de flux (50) comprend un réservoir de mesure vertical (56) doté de deux éléments de senseur de niveau d'eau (51, 52).

7. La méthode de l'une quelconque des revendications précédentes, dans laquelle le volume de non-filtration défini (NV) est inférieur à 10 ml.

8. La méthode de l'une quelconque des revendications précédentes, dans laquelle la membrane de filtre (24) est distalement non supportée mécaniquement.

9. La méthode de l'une quelconque des revendications précédentes, dans laquelle aucun senseur de pression n'est fourni fluidiquement entre la sonde de filtre (20) et la pompe à échantillon (40).

10. La méthode de l'une quelconque des revendications précédentes, dans laquelle le moteur de pompe (46) est un moteur à pas.

11. La méthode de l'une quelconque des revendications précédentes, dans laquelle une mémoire de hauteur hydrostatique (64) pour mémoriser la valeur de hauteur hydrostatique de la pompe (H) entre la pompe à échantillon (40) et la surface de l'eau (40') dans le bassin d'eau (12), dans laquelle la détermination de la valeur de la condition de colmatage du filtre à échantillon (C) comprend la hauteur hydrostatique de la pompe (H).

12. La méthode de l'une quelconque des revendications précédentes, dans laquelle la valeur de condition de colmatage du filtre à échantillon (C) est d'autant meilleure que le rapport est petit entre la valeur des rotations du moteur de filtration (FR) et la valeur des rotations du moteur de non-filtration (NR).

13. La méthode de l'une des revendications précédentes, dans laquelle l'arrangement (10) détermine une valeur de condition de conduite de pompe (CH) sur la base de la valeur de rotations du moteur de non-filtration (NR).
